(19) Europäisches Patentamt
European Patent Office
Office européen des brevets

(11) **EP 0 537 643 B1**

(12) **EUROPÄISCHE PATENTSCHRIFT**

(45) Veröffentlichungstag und Bekanntmachung des Hinweises auf die Patenterteilung:
**05.03.1997 Patentblatt 1997/10**

(51) Int Cl.$^6$: **G02B 5/18**, A61F 2/16, G02C 7/06, G02B 3/08

(21) Anmeldenummer: **92117300.1**

(22) Anmeldetag: **09.10.1992**

(54) **Ophthalmische Linse**

Ophthalmic lens

Lentille ophthalmique

(84) Benannte Vertragsstaaten:
**AT BE CH DE DK ES FR GB GR IE IT LI LU MC NL PT SE**

(30) Priorität: **18.10.1991 DE 4134518**

(43) Veröffentlichungstag der Anmeldung:
**21.04.1993 Patentblatt 1993/16**

(73) Patentinhaber: **Chiron Adatomed Pharmazeutische und Medizintechnische Gesellschaft mbH**
**85609 Dornach (DE)**

(72) Erfinder:
• **Stork, Wilhelm, Dr.**
**W-8510 Fürth (DE)**
• **Streibl, Norbert, Dr.**
**W-8520 Erlangen (DE)**

(74) Vertreter: **Nöth, Heinz, Dipl.-Phys. Patentanwalt, Mozartstrasse 17 80336 München (DE)**

(56) Entgegenhaltungen:
**EP-A- 0 355 230 EP-A- 0 393 639**
**EP-A- 0 470 811 US-A- 4 895 790**

**Beschreibung**

Die Erfindung betrifft eine ophthalmische Linse, in deren optischem Linsenteil auf Zonenradien um die optische Linsenachse diffraktive Feinstrukturelemente vorgesehen sind.

Eine derartige ophthalmische Linse ist aus der EP-A- 0 355 230 bekannt. Durch die diffraktive Feinstruktur wird eine multifokale, insbesondere bifokale Linse geschaffen. Auch in der EP-A-0 470 811 wird eine derartige Linse beschrieben.

Aufgabe der Erfindung ist es, eine ophthalmische Linse der eingangs genannten Art zu schaffen, die eine verringerte Linsendicke aufweist.

Diese Aufgabe wird erfindungsgemäß dadurch gelöst, daß die Linse als refraktive Linse ausgebildet ist, wobei die Feinstrukturelemente derart geformt sind, daß der optische Linsenteil eine monofokale Linse bildet, deren Brechkraft aus einem refraktiven Anteil und einem diffraktiven Anteil zusammengesetzt ist.

Bei einer bevorzugten Ausführungsform weisen die diffraktiven Feinstrukturelemente ein Sägezahnprofil auf, deren Profilhöhe einer Designwellenlänge im grünen Spektralbereich des sichtbaren Lichtes entspricht.

Anhand der Figuren wird die Erfindung noch näher erläutert. Es zeigt:

Fig. 1    eine Periode einer als Oberflächenprofil auf der Linsenoberfläche ausgebildeten zonenförmigen diffraktiven Feinstruktur in Rechteckdesign;

Fig. 2    eine Energieverteilung über die Beugungsordnungen des in Fig. 1 dargestellten Rechteckdesigns;

Fig. 3    eine Tiefenverteilung des in Fig. 1 dargestellten Rechteckdesigns mit einer Apertur von 7 mm, einer Brechzahladdition von +/- 3 dpt auf 20 dpt, entworfen und betrieben bei einer Designwellenlänge von 555 nm:

Fig. 4    eine Tiefenverteilung für das in Fig. 1 dargestellte Rechteckdesign mit Brechzahladdition von +/- 3 dpt auf 20 dpt, entworfen und betrieben bei einer Designwellenlänge von 555 nm, jedoch mit einer Apertur von 3 mm;

Fig. 5    eine Periode eines Sägezahnprofils einer als Oberflächenprofil auf der Linsenoberfläche zonenförmig ausgebildeten diffraktiven Feinstruktur;

Fig. 6    den Wirkungsgrad des in Fig. 5 dargestellten Sägezahndesigns für gleich starke Beugungsordnungen 0, +1;

Fig. 7    eine Energieverteilung über die Beugungsordnungen des in Fig. 5 dargestellten Sägezahndesigns;

Fig. 8    eine Tiefenverteilung des in Fig. 5 dargestellten Sägezahndesigns mit einer Apertur von 7 mm, einer Brechzahladdition von +/- 3 dpt auf 20 dpt, entworfen und betrieben bei einer Designwellenlänge von 555 nm;

Fig. 9    zwei Stufen eines photolithographischen Verfahrens zur Herstellung einer diffraktiven Feinstruktur auf einer Linsenoberfläche, wobei in der ersten Stufe ein Rechteckdesign und in der zweiten Stufe ein stufenförmig angehähertes Sägezahndesign hergestellt wird; und

Fig. 10, 11, 12 Ausführungsbeispiele für ophthalmische Linsen.

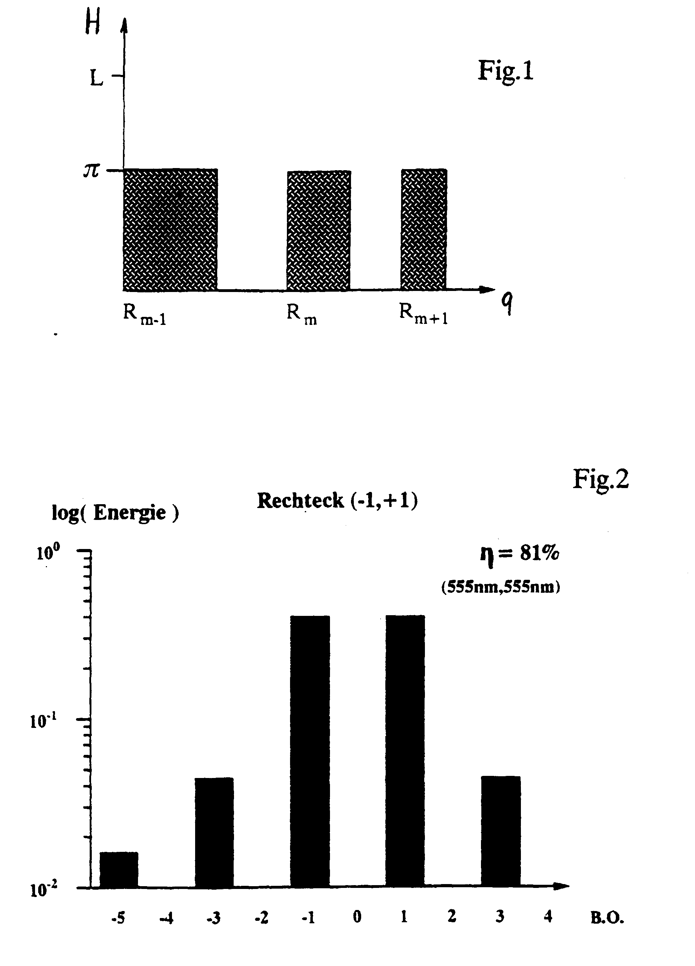

In der Fig. 1 ist die Periode eines Oberflächenprofils eines Rechteckdesigns für eine diffraktive zonenförmige Feinstruktur für eine Intraokularlinse dargestellt. Die Brechkraft der Intraokularlinse beträgt 20 dpt, wobei durch die diffraktiven Feinstrukturelemente, welche in den Zonen des optisch wirksamen Linsenteils vorgesehen sind, eine Änderung um 3 dpt bewirkt wird. Das bedeutet, daß die +1. Beugungsordnung die Brechkraft um 3 dpt verringert und die -1. Beugungsordnung die Brechkraft um 3 dpt erhöht. Bei dem in der Fig. 1 dargestellten Rechteckdesign der diffraktiven Oberflächenfeinstruktur der Linse werden die +1. und die -1. Beugungsordnungen zueinander abgeglichen und in ihrem Wirkungsgrad maximiert. Wie aus Fig. 1 zu ersehen ist, läßt sich das Rechteckprofil für die Oberflächenstruktur durch die Höhe H und das Tastverhältnis q bestimmen. Die Höhe ist auf L=2 $\Pi$ normiert und

die Zonenradien (Rm) zur Bildung der Perioden des Feinstrukturprofils erfüllen die Gleichung $\sqrt{Rm^2 + f^2}$ -f=$\lambda$, wobei m jeweils eine ganze Zahl, beginnend mit l,f der Reziprokwert jeweils eines der beiden Scheitelbrechwerte der Linse und $\lambda$ die Designwellenlänge, welche im grünen Spektralbereich des sichtbaren Lichts liegt, bedeuten.

Durch eine Parametereinstellung q = 0,5 und H = 0,5 lassen sich die Beugungsordnungen -1 und +1 auf einen maximalen Wirkungsgrad $\eta$ von ca. 81 % abgleichen. H = 0,5 entspricht einer halben Wellenlänge Unterschied in der optischen Wellenlänge (aus EP-A-0 355 230 ist dies bekannt).

In der Fig. 2 ist ein Balkendiagramm für die Energieverteilung auf die einzelnen Beugungsordnungen (mit B.O. bezeichnet) dargestellt. Wie sich zeigt, fallen die geradzahligen Beugungsordnungen für das in Fig. 1 dargestellte Rechteckdesign vollständig aus.

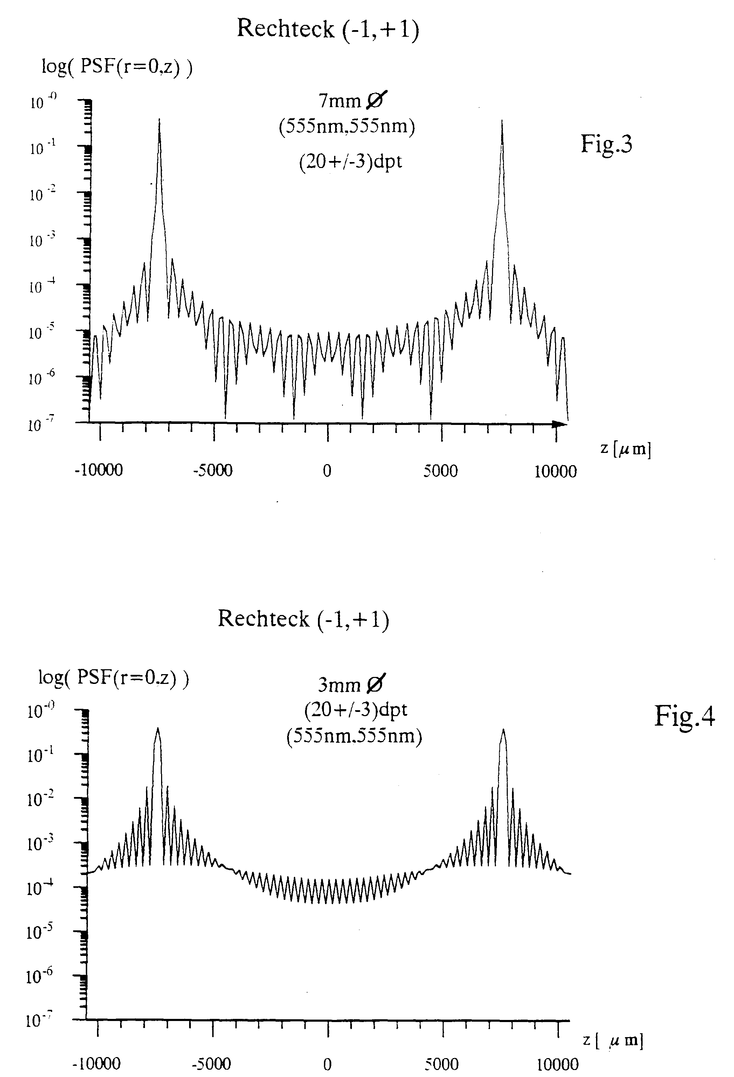

In der Fig. 3 ist die Tiefenverteilung PSF (r = 0,z) für das in Fig. 1 dargestellte Rechteckdesign dargestellt.

Dies ist die Intensitätsverteilung auf der optischen Achse z, woraus sich das longitudinale Auflösungsvermögen, d.h. die Schärfentiefe, ablesen läßt. Die Tiefenverteilung ist logarithmisch aufgetragen, um dem Sehverhalten des Auges möglichst nahezukommen und um auch kleine Effekte deutlich herauszustellen. Die Höhe der Peaks (Foki) entspricht den im Balkendiagramm der Fig. 2 dargestellten Höhen für die -1. und die +1. Beugungsordnungen des Rechteckgitters. Zwischen den beiden Foki gibt es leichte Oszillationen, die jedoch weit unterhalt von 1 Promille liegen. Dieser oszillatorische Untergrund entsteht durch Überlagerung des Lichts aller Zerstreuungskreise am entsprechenden Ort auf der optischen Achse z. Die in der Fig. 3 dargestellte Tiefenverteilung entspricht einer Apertur von 7 mm, d.h. einem Pupillendurchmesser von 7 mm. Dies ist der Durchmesser einer voll geöffneten Pupille.

Bei durchschnittlicher Leuchtdichte beträgt jedoch - bedingt durch die Adaption des Auges - der Durchmesser der Pupille (Apertur) ca. 3 mm.

In der Fig. 4 ist für eine solche Apertur die Tiefenverteilung des in Fig. 1 dargestellten Rechteckdesigns dargestellt. Wie aus Fig. 4 zu ersehen ist, nimmt die Schärfentiefe aufgrund der Aperturverringerung zu.

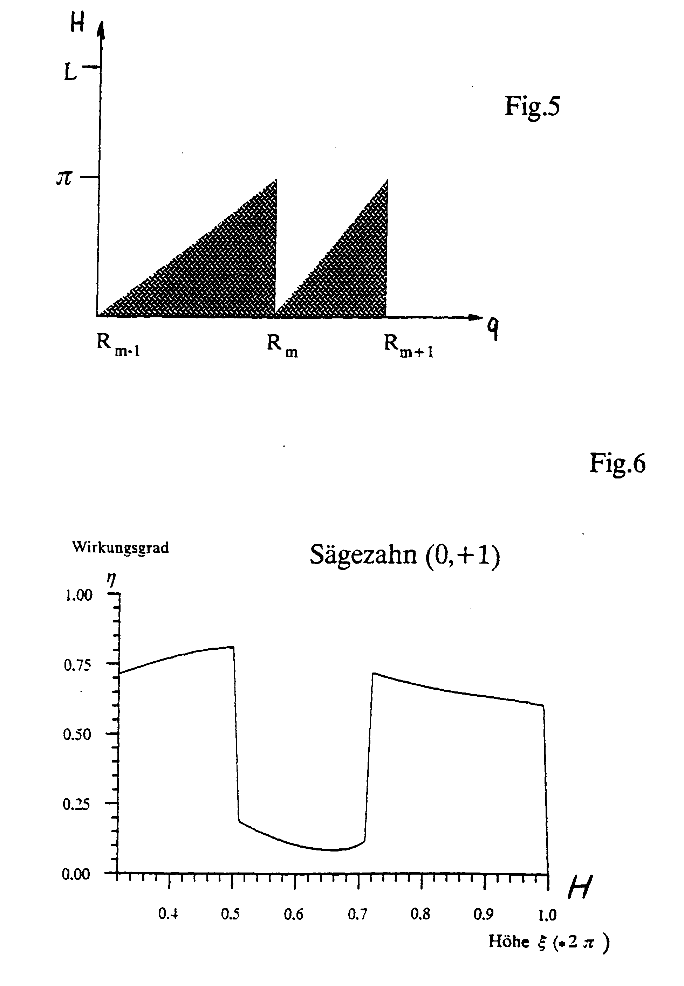

Die Fig. 5 zeigt eine Periode eines Sägezahnprofils für die diffraktive Feinstruktur auf der Linsenoberfläche. Das dargestellte Sägezahndesign gleicht die 0. und die +1. Beugungsordnungen zueinander ab und maximiert diese im Wirkungsgrad. Wie aus der Darstellung der Fig. 5 zu erkennen ist, wird das Sägezahnprofil durch die Höhe des Dreiecks bestimmt. Die Höhe H ist auf L=2 $\Pi$ normiert. Für H = 0,5 sind die Beugungsordnungen 0 und +1 auf einen maximalen Wirkungsgrad von ca. 81 % abgeglichen. Aus EP-A-0 355 230 ist dies bekannt.

In der Fig. 6 ist der Verlauf des Wirkungsgrades bei gleich hellen Beugungsordnungen 0 und +1 in Abhängigkeit von der Höhe H dargestellt. Ein Optimum des Wirkungsgrades $\eta$ von 81 % ergibt sich für eine Röhe H = 0,5. Dies entspricht einer halben Wellenlänge Unterschied in der optischen Wellenlänge (Designwellenlänge). Auch bei einer Höhe H von etwa 0,71 erreicht man einen relativ hohen Wirkungsgrad von etwa 0,74 (74 %). Die Designwellenlänge beträgt 555 nm.

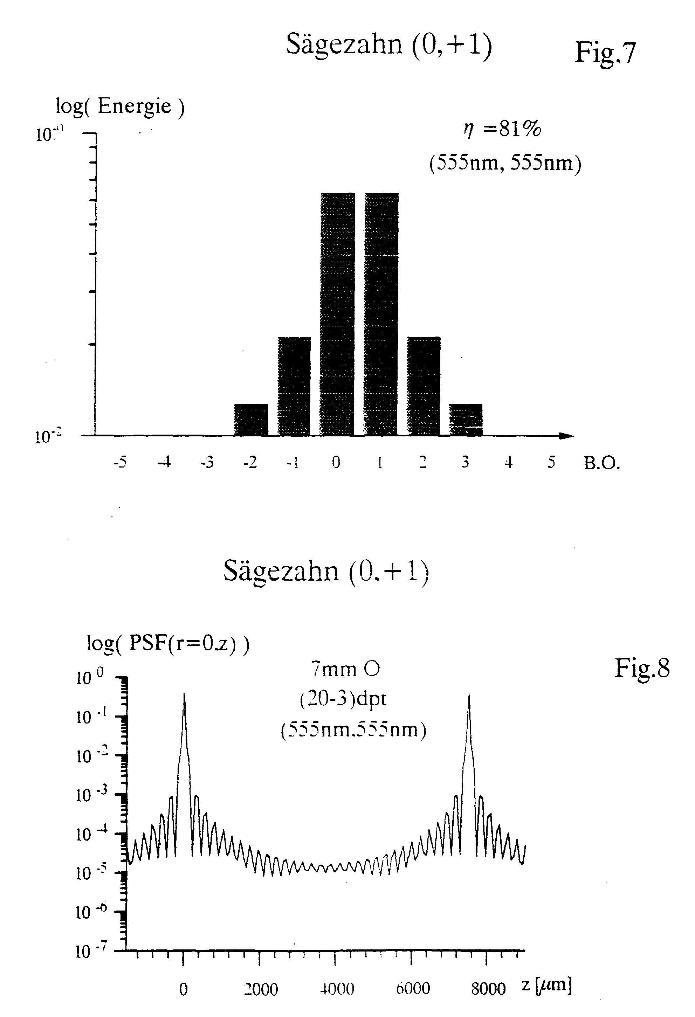

In der Fig. 7 ist die Energieverteilung über die Beugungsordnungen (mit B.O. bezeichnet) des Fig. 5 dargestellten Sägezahnprofils in Form eines Balkendiagramms dargestellt. Die Intensität entlang der optischen Achse z ist durch die in Fig. 8 gezeigte Tiefenverteilung PSF (r = 0,z) Wiedergegeben. Die Höhe der Foki für die 0. und die +1. Beugungsordnung des Sägezahngitters der Fig. 5 entspricht den im Balkendiagramm der Fig. 7 gezeigten Höhen.

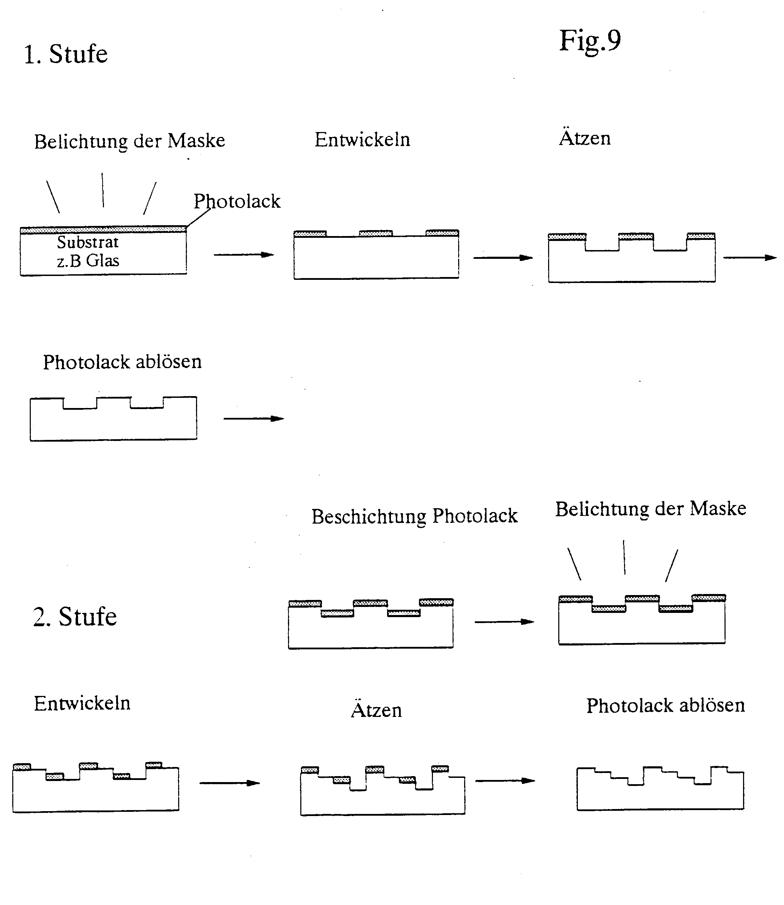

In der Fig. 9 ist ein photolithographisches Verfahren schematisch dargestellt, das bei der Herstellung der diffraktiven Oberflächenfeinstruktur auf der Linse zur Anwendung kommen kann. Die erste Stufe des Herstellungsverfahrens in der Fig. 9 zeigt die Gewinnung einer diffraktiven Feinstruktur mit Rechteckdesign. In der zweiten Stufe ist die Herstellung von mikrofeinen Stufen zur Annäherung der schrägen Rampen für das Sägezahnprofil dargestellt. Es werden hierzu mehrere Maskierungs- und Ätzschritte, wie in der zweiten Stufe dargestellt ist, zur Anwendung gebracht. Das in der zweiten Stufe dargestellte Verfahren kann zur Gewinnung noch feinerer Mikrostufen wiederholt werden. Ein derartiges photolithographisches Verfahren ist aus US-A-4 895 790 bekannt.

Bei der ophthalmischen Linse (Brillenglas, Kontaktlinse, Intraokularlinse) der Erfindung sind die Gewichtungen in der Intensität der beiden Foki so gewählt, daß einer der beiden Foki unterdrückt wird. Dabei wird zur Brechkraft der refraktiven Linse noch eine zusätzliche diffraktive Brechkraft hinzugefügt. Bevorzugt kommt hierbei das Sägezahnprofil für die diffraktive Feinstruktur zur Anwendung, wobei die Profilhöhe bzw. die Differenz der optischen Wellenlänge zwischen Spitze und Tal der Designwellenlänge bevorzugt einer Wellenlänge im grünen Spektralbereich des sichtbaren Lichts entspricht. In diesem Fall wirkt das diffraktive Feinstrukturprofil für die Designwellenlänge als monofokale Linse. Selbst bei Abweichungen von der Designwellenlänge im grünen Spektralbereich, beispielsweise 550 nm, im sichtbaren Bereich (450 - 650 nm) ist die Beugungseffizienz immer noch ca. 80 %, so daß sich Streulichtanteile nicht besonders störend auswirken. Durch die zusätzliche diffraktive Brechkraft erreicht man eine Verringerung der Linsendicke bei gleicher Brechkraft.

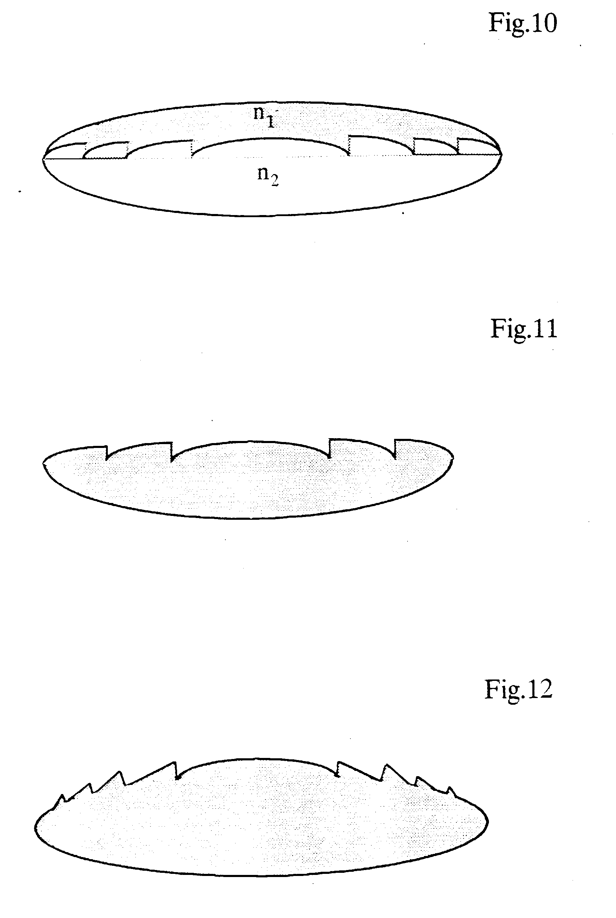

In den Fig. 10 bis 12 sind Ausführungsbeispiele ophthalmischer Linsen, insbesondere Intraokularlinsen, dargestellt. Beim Ausführungsbeispiel der Fig. 10 besteht der Linsenkörper aus zwei Stoffen mit unterschiedlichen Brechungsindizes n1 und n2. Die beiden Linsenteile sind plankonvexe Linsenteile und besitzen an ihren planen Flächen, die aneinander liegen, die diffraktive Struktur, welche schematisch dargestellt ist. Die beiden Linsenteile mit den unterschiedlichen Brechungsindizes sind längs der optischen Achse hintereinander angeordnet.

Bei dem Ausführungsbeispiel der Fig. 11 ist der Linsenkörper plankonvex ausgebildet. Die diffraktive Struktur befindet sich an der planen Seite des Linsenkörpers.

Bei dem in der Fig. 12 dargestellten Ausführungsbeispiel ist der Linsenkörper bikonvex ausgebildet. Die diffraktive Struktur befindet sich auf der einen konvexen Seite des Linsenkörpers.

**Patentansprüche**

1. Ophthalmische Linse, in deren optischem Linsenteil auf Zonenradien um die optische Linsenachse diffraktive Feinstrukturelemente vorgesehen sind, dadurch **gekennzeichnet**, daß die Linse als refraktive Linse ausgebildet ist, wobei die Feinstrukturelemente derart geformt sind,

daß der optische Linsenteil eine monofokale Linse bildet, deren Brechkraft aus einem refraktiven Anteil und einem diffraktiven Anteil zusammengesetzt ist.

2. Ophthalmische Linse nach Anspruch 1, dadurch gekennzeichnet, daß die diffraktiven Feinstrukturelemente ein Sägezahnprofil aufweisen, deren Profilhöhe einer Designwellenlänge im grünen Spektralbereich des sichtbaren Lichtes entspricht.

## Claims

1. An ophthalmic lens in whose optical lens portion diffractive fine structure elements are provided on zone radii around the optical lens axis, characterised in that the lens is in the form of a refractive lens, wherein the fine structure elements are so shaped that the optical lens portion forms a monofocal lens whose refractive power is composed of a refractive component and a diffractive component.

2. An ophthalmic lens according to claim 1 characterised in that the diffractive fine structure elements have a sawtooth profile whose profile height corresponds to a design wavelength in the green spectral range of visible light.

## Revendications

1. Lentille ophtalmique, dans la partie optique de laquelle des éléments microstructurels de diffraction sont prévus sur des rayons zonaux autour de l'axe optique de la lentille, caractérisée en ce que la lentille est réalisée sous la forme d'une lentille réfractive, les éléments microstructurels étant formés de telle sorte que la partie optique de la lentille est une lentille monofocale dont le pouvoir convergent se compose d'une partie réfractive et d'une partie diffractive.

2. Lentille ophtalmique selon la revendication 1, caractérisée en ce que les éléments microstructurels de diffraction présentent un profil en dents de scie dont la hauteur de profil correspond à une longueur d'onde de calcul dans la région verte du spectre de la lumière visible.

Fig.1

Fig.2

Rechteck (-1,+1)

log( Energie )

$\eta = 81\%$

(555nm,555nm)

## Rechteck (-1,+1)

log( PSF(r=0,z) )

7mm ⌀
(555nm,555nm)

(20+/-3)dpt

Fig.3

$10^{-0}$
$10^{-1}$
$10^{-2}$
$10^{-3}$
$10^{-4}$
$10^{-5}$
$10^{-6}$
$10^{-7}$

z [μm]

-10000    -5000    0    5000    10000

## Rechteck (-1,+1)

log( PSF(r=0,z) )

3mm ⌀
(20+/-3)dpt
(555nm,555nm)

Fig.4

$10^{-0}$
$10^{-1}$
$10^{-2}$
$10^{-3}$
$10^{-4}$
$10^{-5}$
$10^{-6}$
$10^{-7}$

z [ μm]

-10000    -5000    0    5000    10000

Fig.5

Fig.6

Wirkungsgrad

Sägezahn (0,+1)

Höhe $\xi$ ($*2\pi$)

Sägezahn (0,+1)          Fig.7

log( Energie )

$\eta = 81\%$

(555nm, 555nm)

Sägezahn (0,+1)

log( PSF(r=0,z) )

7mm O          Fig.8

(20-3)dpt

(555nm,555nm)

1. Stufe                                                         Fig.9

Belichtung der Maske          Entwickeln              Ätzen

Photolack

Substrat
z.B Glas

Photolack ablösen

                              Beschichtung Photolack    Belichtung der Maske

2. Stufe

Entwickeln                    Ätzen                  Photolack ablösen

Fig.10

$n_1$

$n_2$

Fig.11

Fig.12